# EUROPEAN PATENT APPLICATION

(11) **EP 1 897 489 A1**
(43) Date of publication of application: **12.03.2008**
(21) Application number: 06756436.9
(22) Date of filing: 22.05.2006
(51) Int. Cl.: A61B 5/0245

(54) **PULSE WAVE MEASURING INSTRUMENT MANUFACTURABLE AT SUPPRESSED COST**

(30) Priority: 22.06.2005 JP 2005181984
(71) Applicant: Omron Healthcare Co., Ltd., Kyoto 615-0084 (JP)
(72) Inventor: TANABE, Kazuhisa, c/o OMRON HEALTHCARE CO., LTD., Kyoto-shi, Kyoto 615-0084 (JP)
(74) Representative: Wilhelms . Kilian & Partner Patentanwälte
(86) International application number: PCT/JP2006/310130
(87) International publication number: WO 2006/137231

(57) **Abstract**

A pulse wave measuring apparatus includes a pump (9) and a three-port valve (10). The pump (9) includes an exhaust port (91) and an intake port (92), and performs an operation to introduce gas in an air tube (32) from the intake port (92) and discharge the gas into an air tube (30) from the exhaust port (91). The three-port valve (10) performs an operation to selectively connect a cuff (13) to the exhaust port (91) of the pump (9) or connect the cuff (13) to the intake port (92) of the pump (9) and an exhaust valve (7). As a result of this operation, switching between a state that a gas flow path between the air tube (30) and an air tube (31) is established and a gas flow path between the air tube (31) and the air tube (32) is shut off and a state that the gas flow path between the air tube (31) and the air tube (32) is established and the gas flow path between the air tube (30) and the air tube (31) is shut off is made.

## Description

### TECHNICAL FIELD

The present invention relates to a pulse wave measuring apparatus, and more particularly to a pulse wave measuring apparatus manufactured with suppressed cost and achieving lower cost, that adopts a method of increasing pressing against an artery of a living body by a pressure pulse wave sensor by inflating an air bag to which the pressure pulse wave sensor is attached.

### BACKGROUND ART

Japanese Patent Laying-Open No. 2005-95392 (hereinafter, referred to as Patent Document 1) filed previously by the applicant of the subject application discloses a method and a configuration for adjusting a level of pressing by a pressure pulse wave sensor in a pulse wave measuring apparatus measuring a pressure pulse wave of an artery by pressing the pressure pulse wave sensor against the artery of a living body.

Specifically, Patent Document 1 discloses a method of increasing pressing against the artery of the living body by the pressure pulse wave sensor by inflating an air bag, as a configuration that gas is sent from a gas supply source to the air bag (cuff) to which the pressure pulse wave sensor is attached.

Meanwhile, Japanese Patent Laying-Open No. 2004-313409 (Patent Document 2) filed previously by the applicant of the subject application discloses, as an exemplary configuration of a pulse wave measuring apparatus, a configuration adopting a negative pressure pump for keeping a volume of an air bag small by setting a pressure within the air bag forming a part of pressing adjustment means lower than an atmospheric pressure at the time of turn-on of power so that inadvertent protrusion of a pressure pulse wave sensor does not result in failure, in a pulse wave measuring apparatus adopting a method of increasing pressing against an artery of a living body by the pressure pulse wave sensor by inflating the air bag to which the pressure pulse wave sensor as described above is attached.

In the configuration of a conventional pulse wave measuring apparatus as described above, in order to attain a function to avoid failure due to inadvertent protrusion of a pressure pulse wave sensor, for example, a negative pressure pump for suppressing protrusion of the pressure pulse wave sensor is required separately from a pressurizing pump for pressing the pressure pulse wave sensor. In addition, a three-port valve for switching a flow path is required in order to switch between functions of pressurization and pressure reduction.

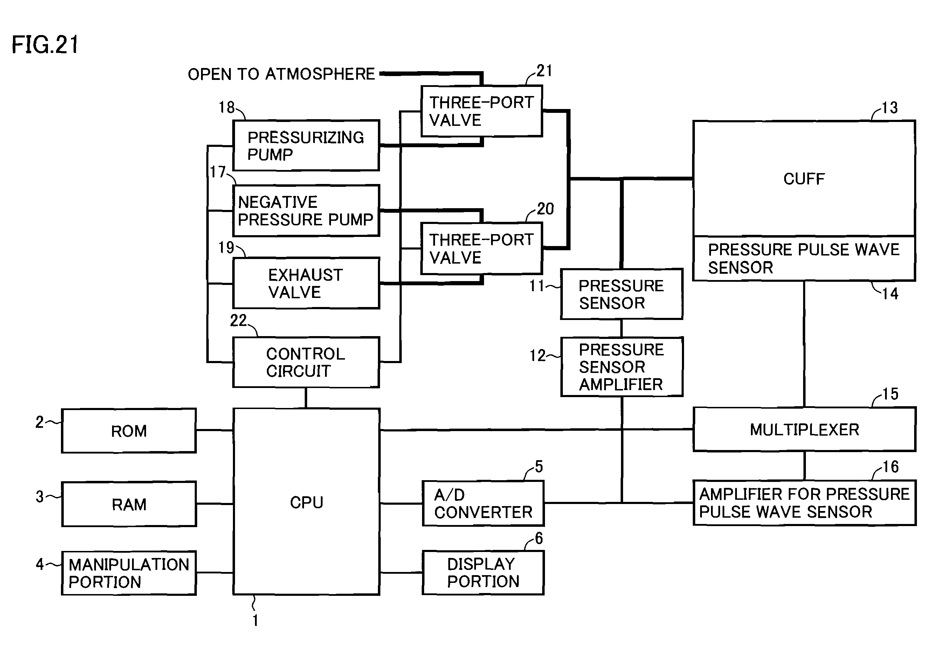

Fig. 21 shows an example of a configuration of a conventional pulse wave measuring apparatus attaining such a function as avoiding failure due to inadvertent protrusion of a pressure pulse wave sensor by adopting a method of increasing pressing against an artery of a living body by a pressure pulse wave sensor by inflating an air bag to which the pressure pulse wave sensor is attached. Referring to Fig. 21, the conventional pulse wave measuring apparatus includes a pressurizing pump 18 for increasing an internal pressure of a cuff (air bag) 13 (hereinafter, referred to as a cuff pressure) and a negative pressure pump 17 for reducing the cuff pressure. Pumps 17 and 18 are connected to cuff 13 through two respective three-port valves 20 and 21 1 selectively connecting, by switching, any one of pressurizing pump 18 and negative pressure pump 17 to cuff 13 in response to a control signal from a control circuit 22.
Patent Document 1: Japanese Patent Laying-Open No. 2005-95392
Patent Document 2: Japanese Patent Laying-Open No. 2004-313409

### DISCLOSURE OF THE INVENTION

### PROBLEMS TO BE SOLVED BY THE INVENTION

Thus, in order to attain such functions, in the conventional pulse wave measuring apparatus, the number of parts of a pressure control system is great and these parts are expensive, which is one factor in a higher price of the pulse wave measuring apparatus.

The present invention was made in view of such problems, and an object of the present invention is to provide a pulse wave measuring apparatus manufactured with suppressed cost and achieving lower cost, that attains a function to avoid failure due to inadvertent protrusion of a pressure pulse wave sensor by adopting a method of increasing pressing against an artery of a living body by a pressure pulse wave sensor by inflating an air bag to which the pressure pulse wave sensor is attached.

### MEANS FOR SOLVING THE PROBLEMS

In order to achieve the above-described object, according to one aspect of the present invention, a pulse wave measuring apparatus includes: a pressure sensor pressed against an artery of a living body; a pressing portion for pressing the pressure sensor; a pulse wave measurement portion measuring a pulse wave produced from the artery based on pressure information output from the pressure sensor in a process of variation in a level of pressing the pressure sensor by the pressing portion; a pressing adjustment portion including a gas accommodation portion and adjusting the level of pressing the pressure sensor by using a pressure of gas in the gas accommodation portion; a supply portion supplying gas to the gas accommodation portion; and a flow path control portion including a valve for taking in and discharging the gas between the gas accommodation portion and outside by opening and for shutting off intake and discharge of the gas between the gas accommodation portion and the outside by closing, and adjusting a gas flow path between the outside and the gas accommodation portion.

An air bag (cuff) to which the pressure sensor is attached and an air tube connected to the cuff represent examples of the gas accommodation portion.
Meanwhile, the pulse wave measurement portion includes a CPU (Central Processing Unit).

In addition, preferably, the pulse wave measuring apparatus further includes an exhaust portion discharging the gas in the gas accommodation portion to the outside, the valve includes a first valve connected to an intake side where the gas outside the supply portion is taken in and a second valve connected to an exhaust side where the gas is discharged to the outside of the exhaust portion, and the flow path control portion adjusts the gas flow path between the outside and the gas accommodation portion by controlling opening/closing of the first valve and the second valve and an operation of the supply portion and the exhaust portion.

A pressurizing pump including an intake port and an exhaust port represents an example of the supply portion, and a negative pressure pump including an intake port and an exhaust port represents an example of the exhaust portion. An exhaust valve which is an electromagnetic valve represents an example of the first valve and the second valve. The exhaust port of the pressurizing pump corresponding to the supply portion and the intake port of the negative pressure pump corresponding to the exhaust portion are connected to the air tube corresponding to a part of the gas accommodation portion.

In addition, a control circuit outputting a control signal from the CPU (Central Processing Unit) controlling the overall pulse wave measuring apparatus to each portion represents an example of the flow path control portion.

More specifically, preferably, in moving the pressure sensor away from a surface of the living body by means of the pressing adjustment portion, the flow path control portion controls the supply portion to stop operation and the exhaust portion to operate and controls the first valve to close and the second valve to open, so that a first flow path from the gas accommodation portion via the exhaust portion and the second valve to the outside is established and the gas in the accommodation portion is discharged.

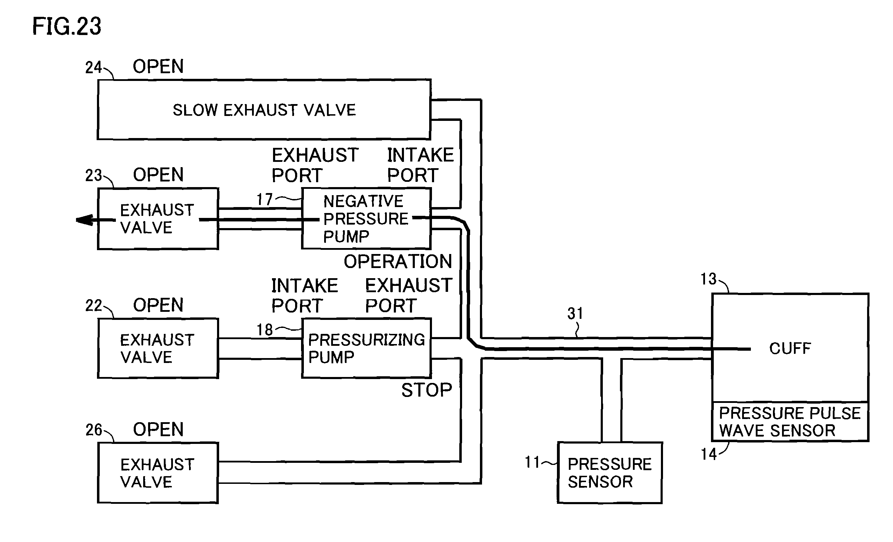

An exemplary first flow path is shown in Fig. 23, in Best Modes for Carrying out the Invention below.

Accordingly, at the time of start of measurement and the like, the gas is discharged from the gas accommodation portion implemented by a cuff or the like, namely the pressure is reduced and a volume is lowered, so that the pressure sensor moves away from the surface of the living body

In addition, more specifically, in pressing the pressure sensor with the pressing portion by means of the pressing adjustment portion, the flow path control portion controls the supply portion to operate and controls the first valve to open and the second valve to close, so that a second flow path from the outside via the first valve and the supply portion to the gas accommodation portion is established and the gas is supplied to the gas accommodation portion.

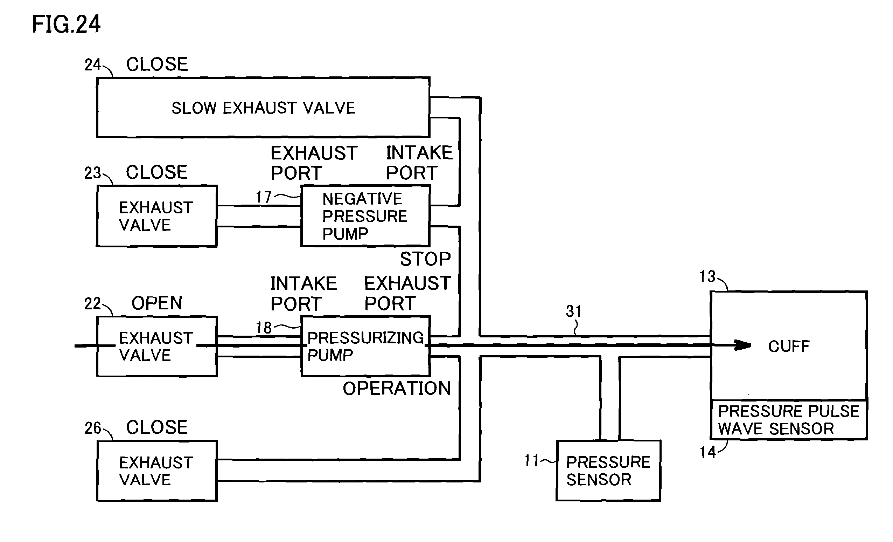

An exemplary second flow path is shown in Fig. 24, in Best Modes for Carrying out the Invention below.

Accordingly, when measurement is started, the gas is supplied to the gas accommodation portion implemented by a cuff or the like, namely the pressure is increased and the volume is increased, so that the pressure sensor is pressed against the surface of the living body.

Moreover, in pressing the pressure sensor with the pressing portion by means of the pressing adjustment portion, preferably, the flow path control portion controls the exhaust portion to operate.

Accordingly, when the gas is supplied to the gas accommodation portion implemented by a cuff or the like and the pressure is increased, the gas in the gas accommodation portion is discharged from the intake port side to the exhaust port side of the exhaust portion implemented by a negative pressure pump or the like, so that the pressure on the exhaust port side of the exhaust portion can be higher than the pressure on the intake port side. Thus, where use of an exhaust pump under such a condition that the pressure on the intake port side is lower than the pressure on the exhaust port side is recommended, the exhaust pump can be adapted to such usage.

In addition, more specifically, preferably, the pulse wave measuring apparatus further includes a slow exhaust portion discharging the gas in the gas accommodation portion to the outside while adjusting an amount of discharge, and in adjusting the level of pressing the pressure sensor by means of the pressing adjustment portion, the flow path control portion controls the supply portion and the exhaust portion to stop operation and the slow exhaust portion to operate and controls the first valve and the second valve to close, so that a third flow path from the gas accommodation portion via the slow exhaust portion to the outside is established and the gas in the gas accommodation portion is discharged.

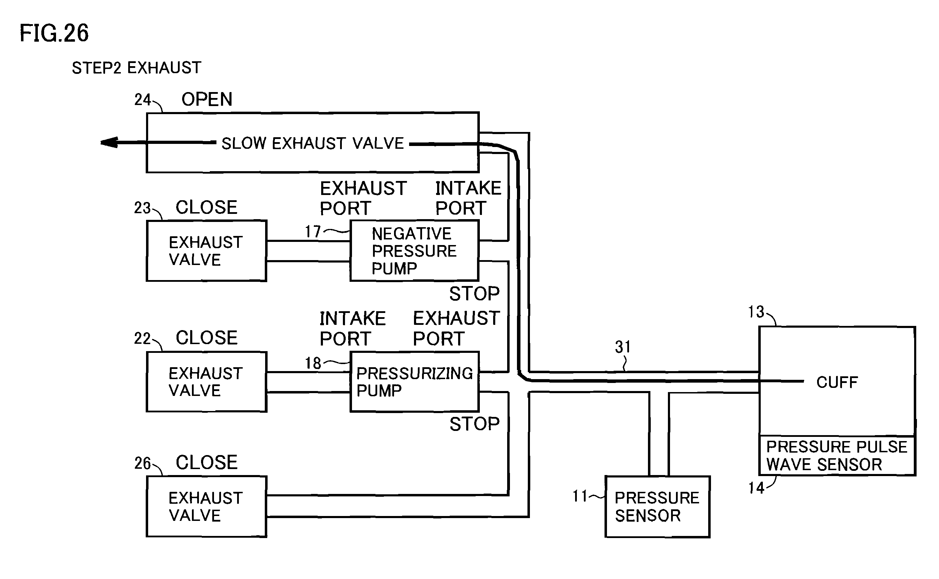

An exemplary third flow path is shown in Fig. 26, in Best Modes for Carrying out the Invention below.

A slow exhaust valve capable of operating to gradually discharge the gas from the exhaust port to the atmosphere while adjusting an amount of discharge in response to a control signal represents an example of the slow exhaust portion. As a result of operation of the slow exhaust portion implemented by a slow exhaust valve or the like, the internal pressure of the gas accommodation portion is gradually reduced and the level of pressing the pressure sensor is gradually lowered.

In addition, more preferably, the flow path control portion repeats establishment of the third flow path and closing of the gas accommodation portion until an internal pressure of the gas accommodation portion attains to a prescribed pressure.

Accordingly, the internal pressure of the gas accommodation portion attains to a prescribed pressure, and the level of pressing the pressure sensor can be optimized.

In addition, more specifically, preferably, the valve further includes a third valve connected to the gas accommodation portion, and in releasing the pressure sensor from the living body by means of the pressing adjustment portion, the flow path control portion controls the supply portion and the exhaust portion to stop operation and controls the first valve and the second valve to close and the third valve to open, so that a fourth flow path from the gas accommodation portion via the third valve to the outside is established and the gas in the gas accommodation portion is discharged.

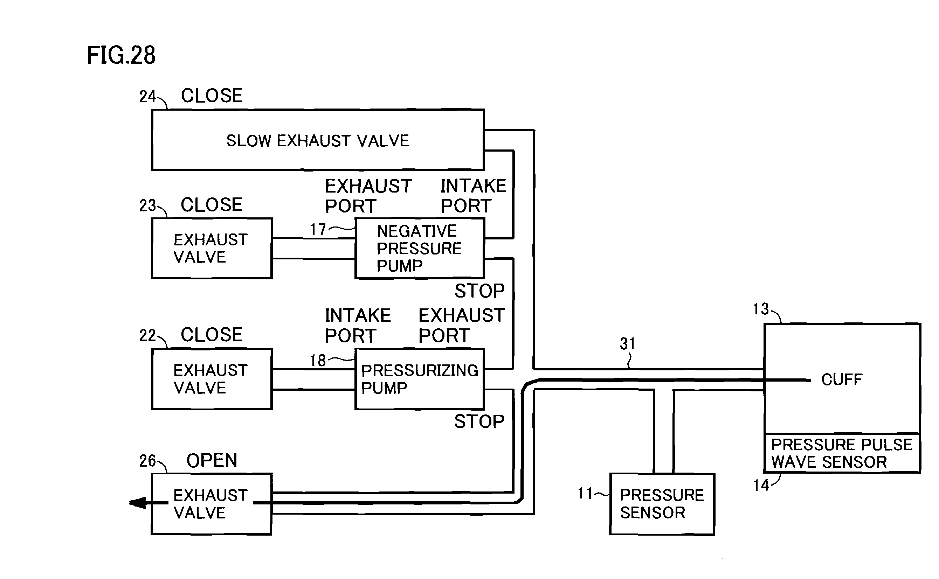

An exemplary fourth flow path is shown in Fig. 28, in Best Modes for Carrying out the Invention below.

Accordingly, when measurement ends, the gas is rapidly discharged from the gas accommodation portion implemented by a cuff or the like, namely the pressure is reduced and a volume is rapidly lowered, so that the pressure sensor rapidly moves away from the surface of the living body.

Alternatively, preferably, the gas accommodation portion includes a first gas accommodation portion, a second gas accommodation portion and a third gas accommodation portion having a prescribed capacity, the pressing adjustment portion adjusts the level of pressing the pressure sensor by using the pressure of the gas in the first gas accommodation portion, the supply portion is provided between the second gas accommodation portion and the third gas accommodation portion and supplies the gas to the second gas accommodation portion by taking in the gas in the third gas accommodation portion and discharging the gas to the second gas accommodation portion, the valve includes a first valve for taking in and discharging the gas between the third gas accommodation portion and the outside by opening and for shutting off intake and discharge of the gas between the third gas accommodation portion and the outside by closing, and the flow path control portion further includes a switching portion switching between a first connection state where the first gas accommodation portion is connected to an exhaust side of the supply portion via the second gas accommodation portion and a second connection state where the first gas accommodation portion is connected to an intake side of the supply portion via the third gas accommodation portion, and adjusts a gas flow path among the first gas accommodation portion, the second gas accommodation portion, and the third gas accommodation portion.

A three-port valve, more specifically a three-port valve having a first port connected to the first gas accommodation portion, for taking in and discharging the gas, a second port connected to the second gas accommodation portion, for taking in and discharging the gas, and a third port connected to the third gas accommodation portion, for taking in and discharging the gas, represents an example of the switching portion

A pump including an intake port and an exhaust port represents an example of the supply portion.

An exhaust valve represents an example of the first valve.

A cuff and an air tube connected to the cuff represent examples of the first gas accommodation portion. A first port of the three-port valve corresponding to the switching portion is connected to the air tube corresponding to the first gas accommodation portion.

An air tube represents an example of the second gas accommodation portion and the third gas accommodation portion. An exhaust port of the pump corresponding to the supply portion and a second port of the three-port valve corresponding to the switching portion are connected to the air tube corresponding to the second gas accommodation portion. An intake port of the pump corresponding to the supply portion, a third port of the three-port valve corresponding to the switching portion, and an exhaust valve corresponding to the first valve are connected to the air tube corresponding to the third gas accommodation portion.

A control circuit outputting a control signal from the CPU controlling the overall pulse wave measuring apparatus to each portion represents an example of the flow path control portion, and the control signal controls connection of each port of the three-port valve corresponding to the switching portion, the operation of the pump corresponding to the supply portion, and opening/closing of the exhaust valve corresponding to the first valve.

More specifically, preferably, in releasing the pressure sensor from a surface of the living body by means of the pressing adjustment portion, the flow path control portion controls the three-port valve to connect the first port to the third port to attain the second connection state, controls the supply portion to operate, and controls the first valve to close, so that a first flow path from the first gas accommodation portion via the third gas accommodation portion to the second gas accommodation portion is established and the gas in the first gas accommodation portion is discharged.

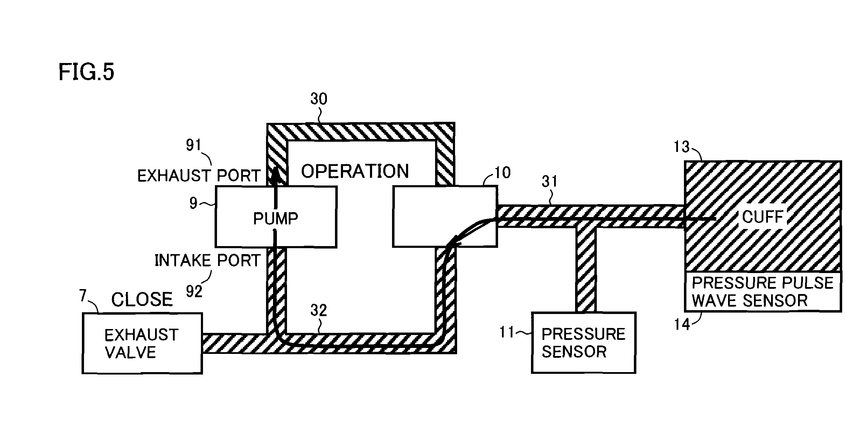

An exemplary first flow path is shown in Fig. 5, in Best Modes for Carrying out the Invention below.

Accordingly, at the time of start of measurement and the like, the gas is discharged from the first gas accommodation portion implemented by a cuff or the like, namely the pressure is reduced and a volume is lowered, so that the pressure sensor moves away from the surface of the living body.

In addition, more specifically, preferably, in pressing the pressure sensor with the pressing portion by means of the pressing adjustment portion, the flow path control portion controls the three-port valve to connect the first port to the second port to attain the first connection state, controls the supply portion to operate, and controls the first valve to open, so that a second flow path from the outside via the third gas accommodation portion and the second gas accommodation portion to the first gas accommodation portion is established and the gas is supplied to the first gas accommodation portion.

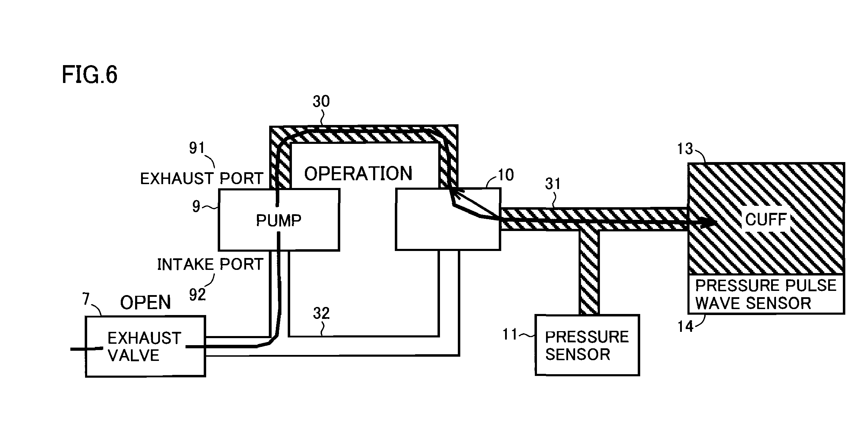

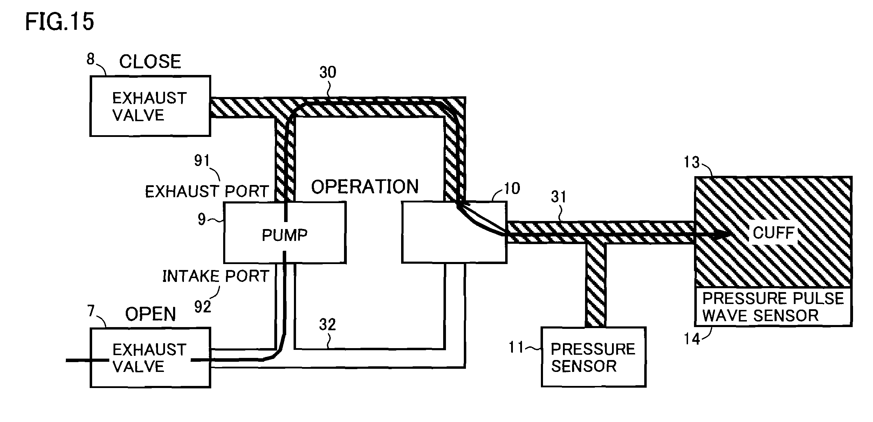

An exemplary second flow path is shown in Fig. 6 or 15, in Best Modes for Carrying out the Invention below.

Accordingly, when measurement is started, the gas is supplied to the first gas accommodation portion implemented by a cuff or the like, namely the pressure is increased and the volume is increased, so that the pressure sensor is pressed against the surface of the living body.

In addition, more specifically, preferably, in adjusting the level of pressing the pressure sensor by means of the pressing adjustment portion, the flow path control portion controls the three-port valve to connect the first port to the second port to attain the first connection state, controls the supply portion to stop the operation, and controls the first valve to open, so that a third flow path from the third gas accommodation portion to the outside is established and an internal pressure of the third gas accommodation portion attains to an atmospheric pressure, and thereafter, after closing the third gas accommodation portion by controlling the first valve to close, the flow path control portion controls the three-port valve to connect the first port to the third port to attain the second connection state and controls the supply portion to stop the operation, so that a fourth flow path from the first gas accommodation portion to the third gas accommodation portion is established and an internal pressure in the first gas accommodation portion is reduced.

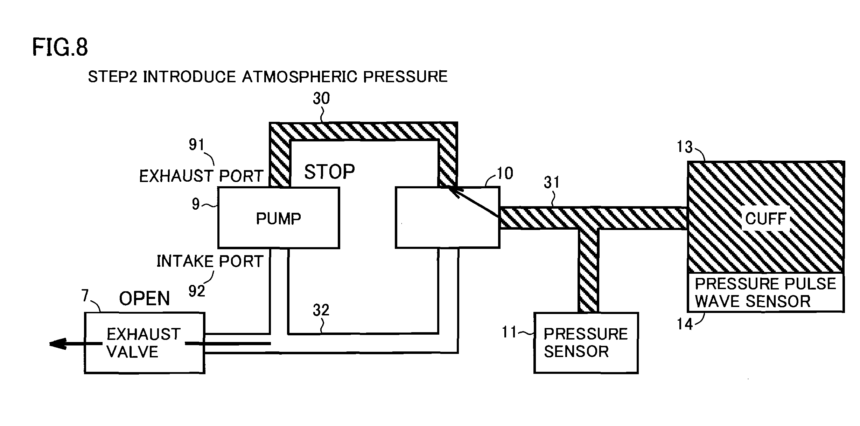

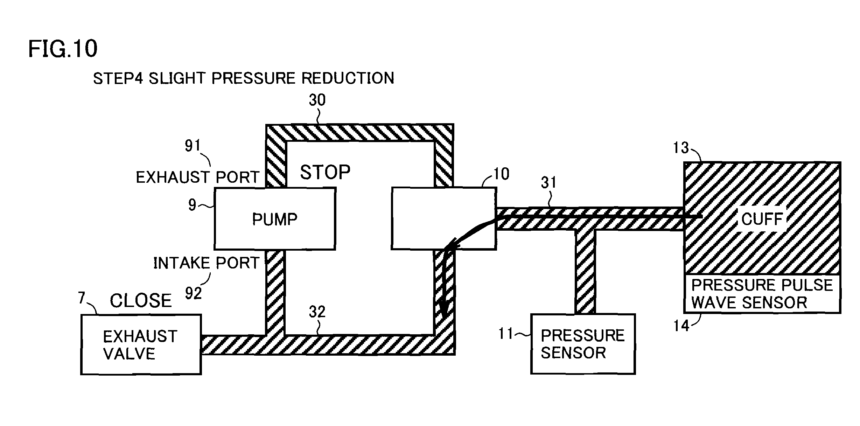

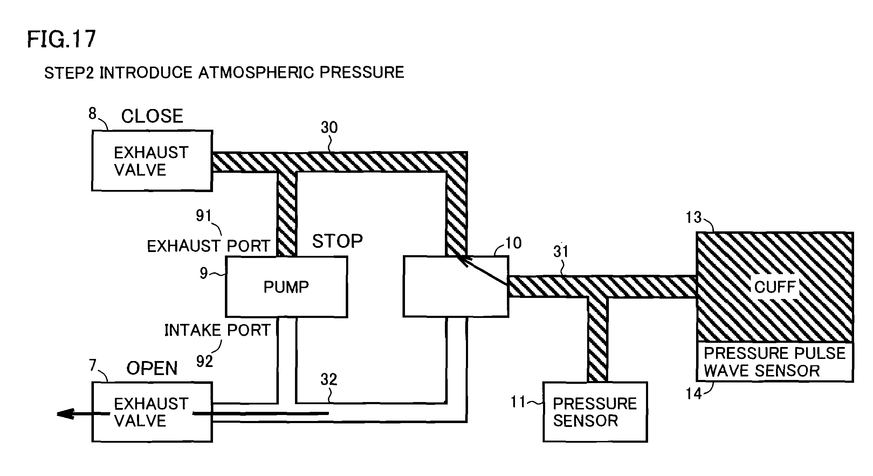

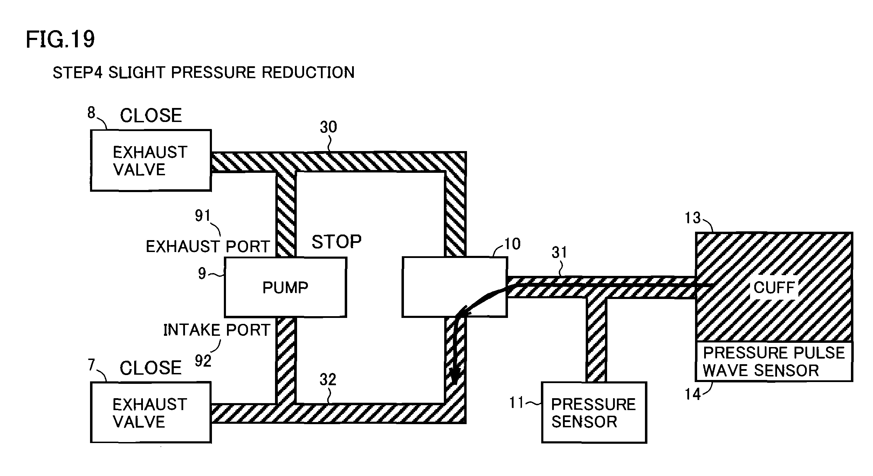

An exemplary third flow path is shown in Fig. 8 or 17, in Best Modes for Carrying out the Invention below, and an exemplary fourth flow path is shown in Fig. 10 or 19, in Best Modes for Carrying out the Invention below.

Reduction in the internal pressure of the third gas accommodation portion to the atmospheric pressure produces difference in the pressure between the first gas accommodation portion and the third gas accommodation portion, and thereafter the fourth flow path is established. Then, the internal pressure of the first gas accommodation portion becomes equal to that of the third gas accommodation portion, the internal pressure of the first gas accommodation portion is reduced, and the level of pressing the pressure sensor is lowered.

In addition, preferably, the flow path control portion repeats establishment of the third flow path, closing of the third gas accommodation portion, and establishment of the fourth flow path until the internal pressure of the first gas accommodation portion attains to a prescribed pressure.

Accordingly, the internal pressure of the first gas accommodation portion attains to a prescribed pressure, and the level of pressing the pressure sensor can be optimized.

In addition, more specifically, preferably, in releasing the pressure sensor from the living body by means of the pressing adjustment portion, the flow path control portion controls the three-port valve to connect the first port to the third port to attain the second connection state, controls the supply portion to stop the operation, and controls the first valve to open, so that a fifth flow path from the first gas accommodation portion via the third gas accommodation portion to the outside is established and the gas in the first gas accommodation portion is discharged.

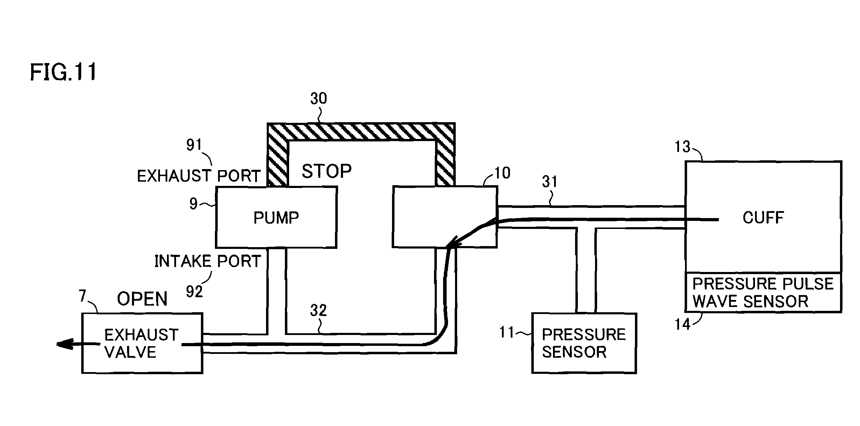

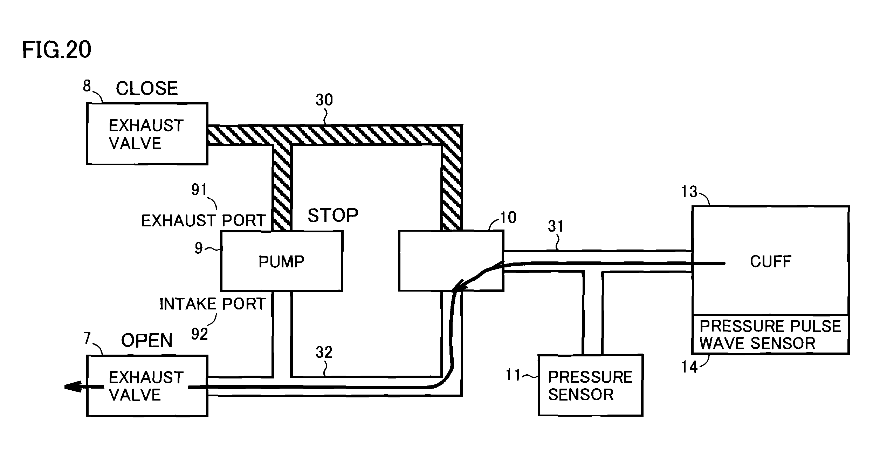

An exemplary fifth flow path is shown in Fig. 11 or 20, in Best Modes for Carrying out the Invention below.

Accordingly, when measurement ends, the gas is rapidly discharged from the first gas accommodation portion implemented by a cuff or the like, namely the pressure is reduced and a volume is rapidly lowered, so that the pressure sensor rapidly moves away from the surface of the living body.

Alternatively, preferably, the valve further includes a second valve for taking in and discharging the gas between the second gas accommodation portion and the outside by opening and for shutting off intake and discharge of the gas between the second gas accommodation portion and the outside by closing, and in moving the pressure sensor away from a surface of the living body by means of the pressing adjustment portion, the flow path control portion controls the three-port valve to connect the first port to the third port to attain the second connection state, controls the supply portion to operate, controls the first valve to close, and controls the second valve to open, so that a sixth flow path from the first gas accommodation portion via the third gas accommodation portion and the second gas accommodation portion to the outside is established and the gas in the first gas accommodation portion is discharged.

An exhaust valve represents an example of the second valve, and the exhaust valve is connected to the air tube corresponding to the second gas accommodation portion, as well as to the exhaust port of the pump corresponding to the supply portion and the second port of the three-port valve corresponding to the switching portion.

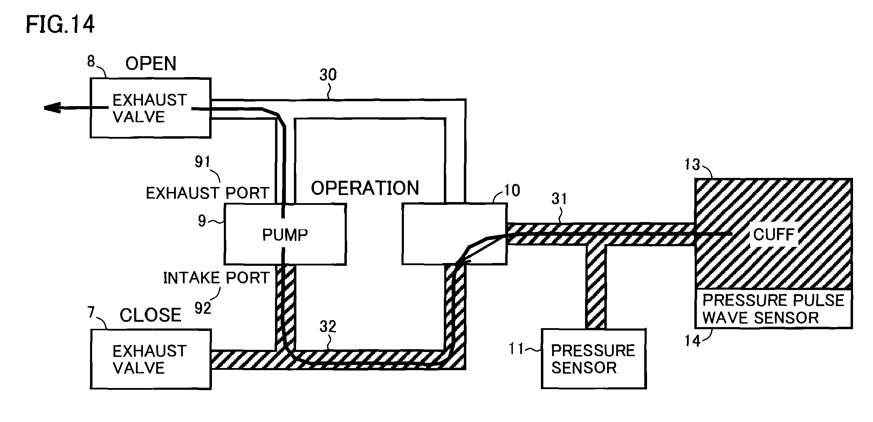

An exemplary sixth flow path is shown in Fig. 14, in Best Modes for Carrying out the Invention below.

Accordingly, at the time of start of measurement and the like, the gas is discharged from the first gas accommodation portion implemented by a cuff or the like to the outside, namely the pressure is reduced and a volume is lowered, so that the pressure sensor moves away from the surface of the living body.

### EFFECTS OF THE INVENTION

With the configuration above, the pulse wave measuring apparatus according to the present invention can realize the configuration for attaining a function to avoid failure due to inadvertent protrusion of a pressure pulse wave sensor with a smaller number of components than in a conventional apparatus and/or the configuration for obtaining a reference offset value for the pressure pulse wave sensor. Alternatively, with a simplified structure and with the use of an inexpensive member for a component, the pulse wave measuring apparatus according to the present invention realizes the configuration for attaining a function to avoid failure due to inadvertent protrusion of a pressure pulse wave sensor and/or the configuration for obtaining a reference offset value for the pressure pulse wave sensor. Therefore, manufacturing cost of the pulse wave measuring apparatus is suppressed and cost reduction can be achieved.

### BRIEF DESCRIPTION OF THE DRAWINGS

Fig. 1 illustrates one example of a configuration of a pulse wave measuring apparatus according to a first embodiment.
Fig. 2 illustrates a graph of variation in a level of pressing a pressure pulse wave sensor in pulse wave measurement in the present embodiment.
Fig. 3 is a flowchart showing one example of measurement sequence of the pulse wave measuring apparatus according to the present embodiment.
Fig. 4 illustrates an operation in step S3 of each portion of the pulse wave measuring apparatus according to the first embodiment.
Fig. 5 illustrates an operation of each portion of the pulse wave measuring apparatus according to the first embodiment and a gas flow path in step S5.
Fig. 6 illustrates an operation of each portion of the pulse wave measuring apparatus according to the first embodiment and a gas flow path in step S7.
Fig. 7 illustrates an operation of each portion of the pulse wave measuring apparatus according to the first embodiment and a gas flow path in STEP 1 of step S9.
Fig. 8 illustrates an operation of each portion of the pulse wave measuring apparatus according to the first embodiment and a gas flow path in STEP 2 of step S9.
Fig. 9 illustrates an operation of each portion of the pulse wave measuring apparatus according to the first embodiment and a gas flow path in STEP 3 of step S9.
Fig. 10 illustrates an operation of each portion of the pulse wave measuring apparatus according to the first embodiment and a gas flow path in STEP 4 of step S9.
Fig. 11 illustrates an operation of each portion of the pulse wave measuring apparatus according to the first embodiment and a gas flow path in step S 13.
Fig. 12 illustrates one example of a configuration of a pulse wave measuring apparatus according to a second embodiment.
Fig. 13 illustrates an operation in step S3 of each portion of the pulse wave measuring apparatus according to the second embodiment.
Fig. 14 illustrates an operation of each portion of the pulse wave measuring apparatus according to the second embodiment and a gas flow path in step S5.
Fig. 15 illustrates an operation of each portion of the pulse wave measuring apparatus according to the second embodiment and a gas flow path in step S7.
Fig. 16 illustrates an operation of each portion of the pulse wave measuring apparatus according to the second embodiment and a gas flow path in STEP 1 of step S9.
Fig. 17 illustrates an operation of each portion of the pulse wave measuring apparatus according to the second embodiment and a gas flow path in STEP 2 of step S9.
Fig. 18 illustrates an operation of each portion of the pulse wave measuring apparatus according to the second embodiment and a gas flow path in STEP 3 of step S9.
Fig. 19 illustrates an operation of each portion of the pulse wave measuring apparatus according to the second embodiment and a gas flow path in STEP 4 of step S9.
Fig. 20 illustrates an operation of each portion of the pulse wave measuring apparatus according to the second embodiment and a gas flow path in step S13
Fig. 21 illustrates one example of a configuration of a conventional pulse wave measuring apparatus.
Fig. 22 illustrates one example of a configuration of a pulse wave measuring apparatus according to a third embodiment.
Fig. 23 illustrates an operation of each portion of the pulse wave measuring apparatus according to the third embodiment and a gas flow path in step S5.
Fig. 24 illustrates an operation of each portion of the pulse wave measuring apparatus according to the third embodiment and a gas flow path in step S7.
Fig. 25 illustrates an operation of each portion of the pulse wave measuring apparatus according to the third embodiment and a gas flow path in STEP 1 of step S9.
Fig. 26 illustrates an operation of each portion of the pulse wave measuring apparatus according to the third embodiment and a gas flow path in STEP 2 of step S9.
Fig. 27 illustrates an operation of each portion of the pulse wave measuring apparatus according to the third embodiment and a gas flow path in STEP 3 of step S9.
Fig. 28 illustrates an operation of each portion of the pulse wave measuring apparatus according to the third embodiment and a gas flow path in step S 13.

### DESCRIPTION OF THE REFERENCE SIGNS

1 CPU; 2 ROM; 3 RAM; 4 manipulation portion; 5 A/D converter; 6 display portion; 7, 8 exhaust valve; 9 pump; 10 three-port valve; 11 pressure sensor; 12 pressure sensor amplifier; 13 cuff; 14 pressure pulse wave sensor; 15 multiplexer; 16 amplifier for pressure pulse wave sensor; 17 negative pressure pump; 18 pressurizing pump; 19, 22, 23, 26 exhaust valve; 20, 21 three-port valve; 22 control circuit; 24 slow exhaust valve; 30, 31, 32 air tube; 91 exhaust port; and 92 intake port.

### BEST MODES FOR CARRYING OUT THE INVENTION

An embodiment of the present invention will be described hereinafter with reference to the drawings. In the description below, the same parts and components have the same reference characters allotted. Their label and function are also identical.

### [First Embodiment]

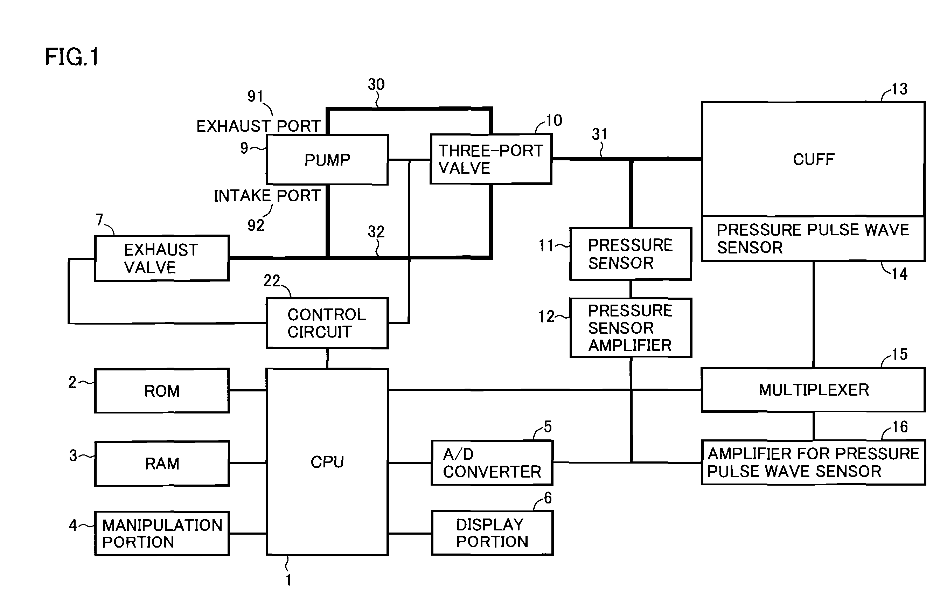

Fig. 1 illustrates one example of a configuration of a pulse wave measuring apparatus according to a first embodiment.

Referring to Fig. 1, the pulse wave measuring apparatus according to the first embodiment includes, as configuration for a control system, a CPU (Central Processing Unit) 1 controlling the overall pulse wave measuring apparatus, a ROM (Read Only Memory) 2 storing data or a program for control, a RAM (Random Access Memory) 3, a manipulation portion 4 provided to be externally operable and operated for input of various types of information, an A/D converter 5 for A/D conversion of a measurement signal, a control circuit 22, and a display portion 6 constituted of an LED (Light Emitting Diode), an LCD (Liquid Crystal Display), or the like for externally outputting various types of information such as a result of pulse wave measurement.

Manipulation portion 4 inputs an operation signal based on user operation to CPU 1, and CPU 1 reads and executes a program through access in response to the input operation signal and sends a control signal to control circuit 22.

In addition, the pulse wave measuring apparatus includes, as configuration for a measurement system, a cuff 13 implemented by an air bag to which a pressure pulse wave sensor 14 is attached, a pressure sensor 11 and a pressure sensor amplifier 12 connected to cuff 13 via an air tube 31, a multiplexer 15 and an amplifier 16 for pressure pulse wave sensor selectively outputting a voltage signal from pressure pulse wave sensor 14, a three-port valve 10 connected to cuff 13 via air tube 31, a pump 9 connected to three-port valve 10 via an air tube 30, and an exhaust valve 7 connected to three-port valve 10 and to pump 9 via an air tube 32.

A semiconductor pressure sensor including a plurality of sensor elements arranged at prescribed intervals in one direction on a semiconductor chip composed of monocrystalline silicon or the like represents an example of pressure pulse wave sensor 14. Pressure pulse wave sensor 14 is pressed against a measurement position such as a wrist of a subject during measurement, with a pressure from cuff 13. In such a state, pressure pulse wave sensor 14 detects a pulse wave of the subject via a radial artery. Pressure pulse wave sensor 14 outputs a voltage signal variable in correspondence with a pressure value of the pulse wave and inputs the voltage signal to multiplexer 15 for each channel of each sensor element. The voltage signal input to multiplexer 15 is amplified in amplifier 16 for pressure pulse wave sensor up to a prescribed range, and selectively input to A/D converter 5 for each voltage signal output by the sensor element.

Pressure sensor 11 measures a cuff pressure equal to the pressure in connected air tube 31, and inputs a voltage signal output as a result of measurement to pressure sensor amplifier 12. The voltage signal input to pressure sensor amplifier 12 is amplified up to a prescribed range and input to A/D converter 5. Alternatively, a capacitive type pressure sensor constituted of a single electrode pair or a plurality of electrode pairs arranged one-dimensionally or two-dimensionally may represent another example of pressure pulse wave sensor 14. In this case, variation in the capacitance in correspondence with a pressure value of the pulse wave is converted to variation in a voltage by amplifier 16 for pressure pulse wave sensor through multiplexer 15, and selectively input to A/D converter 5 for each sensor element.

A/D converter 5 converts a voltage signal which is an analog signal input from multiplexer 15 and a voltage signal which is an analog signal input from pressure sensor amplifier 12 to digital information, and inputs the digital information to CPU 1.

CPU 1 attains a function as a pulse wave measurement portion by executing a program stored in ROM 2, calculates a pulse wave based on the voltage signal converted to digital information, and causes display portion 6 to display the result as a measurement result.

Control circuit 22 connected to exhaust valve 7, pump 9, and three-port valve 10 outputs a control signal to each portion.

Pump 9 includes an exhaust port 91 and an intake port 92, and operates to introduce gas in air tube 32 from intake port 92 and discharge the gas from exhaust port 91 to air tube 30 in response to a control signal input from control circuit 22. Exhaust valve 7 is connected to air tube 32 as well as to intake port 92 side of pump 9 and three-port valve 10, and operates to shut off/establish a flow path between the external gas and air tube 32 in response to the control signal input from control circuit 22.

Three-port valve 10 operates to selectively connect cuff 13 to exhaust port 91 of pump 9 or connect cuff 13 to intake port 92 of pump 9 and exhaust valve 7 in response to the control signal input from control circuit 22. As a result of this operation, switching between a state that the gas flow path between air tube 30 and air tube 31 is established and the gas flow path between air tube 31 and air tube 32 is shut off and a state that the gas flow path between air tube 31 and air tube 32 is established and the gas flow path between air tube 3 0 and air tube 31 is shut off is made.

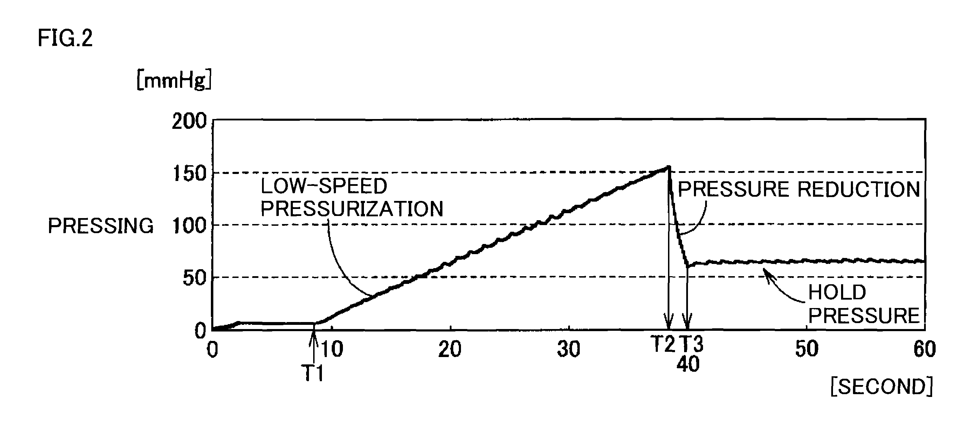

Fig. 2 illustrates a graph of variation in a level of pressing pressure pulse wave sensor 14 in pulse wave measurement in the embodiment. The ordinate of the graph represents a level of pressing, while the abscissa represents lapse of time. Referring to Fig. 5, when measurement is started at time T1, pressure pulse wave sensor 14 placed directly over the artery is pressed against the living body with increase in the cuff pressure. As the cuff pressure caused by the pressing force gradually increases, pressure pulse wave sensor 14 increasingly presses the artery against a radius and the artery is flattened. While pressure pulse wave sensor 14 is pressed against the living body at an optimal pressure, the artery is in a flattened state (tonometry state) where the pulse wave originating from fluctuation in an intra-arterial pressure can accurately be measured. When the level of pressing by cuff 13 is raised, the artery is further flattened and finally squeezed. In the process above, CPU 1 determines an optimal pressure based on variation in a waveform of a pulse wave signal detected by pressure pulse wave sensor 14. During measurement, the level of pressing is lowered to the optimal pressure from time T2 at which the level of pressing attained to the optimal pressure or higher, and the optimal pressure is held after time T3 when CPU 1 has determined that the optimal pressure was attained. The pulse wave signal detected by pressure pulse wave sensor 14 while the optimal pressure is held is converted to pulse wave data by A/D converter 5 and provided to CPU 1, where the pulse wave data is subjected to prescribed processing such as calculation of an index, and the result of processing is displayed on display portion 6.

When measurement ends, the cuff pressure is once rapidly reduced to an atmospheric pressure. The pulse wave measuring apparatus thus measures the pulse wave in the pressing sequence above.

Here, a time period for lowering the pressing level to the optimal pressure (time T2 to T3) is short, and the pressing force is reduced by several tens of mmHg in this short period of time and also further lowered by several mmHg for setting to the optimal pressure. Here, the pressing force is finely adjusted.

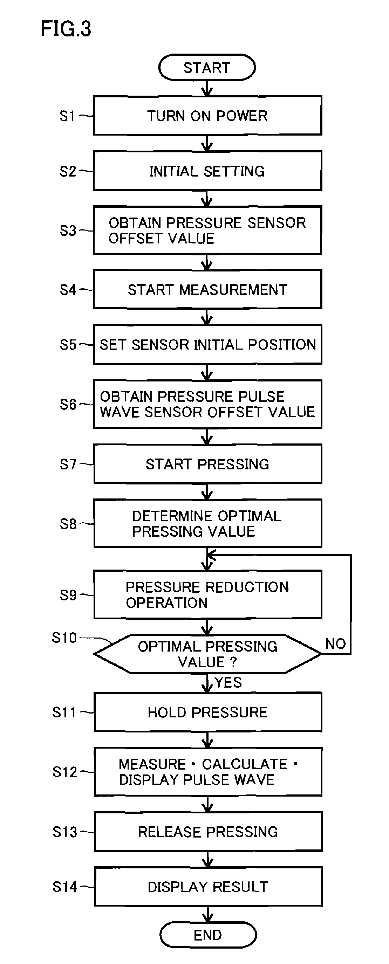

Fig. 3 is a flowchart showing one example of measurement sequence of the pulse wave measuring apparatus according to the present embodiment. The processing shown in the flowchart in Fig. 3 is performed in such a manner that CPU 1 accesses ROM 2 and reads and executes a program.

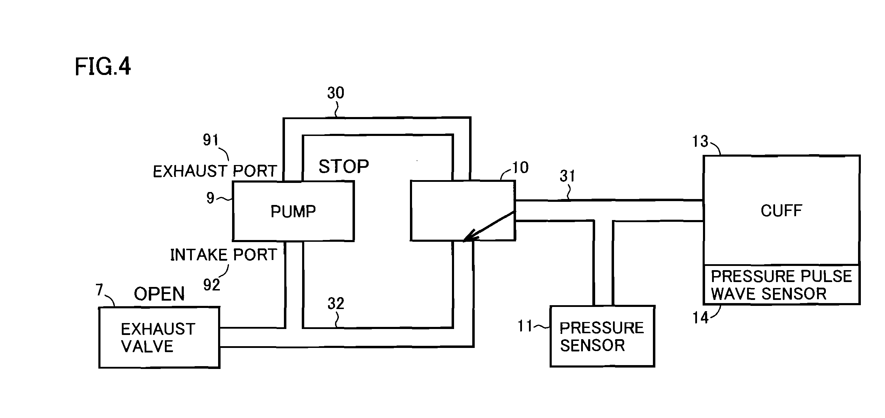

Meanwhile, Figs. 4 to 11 are schematic diagrams illustrating a main portion of the pulse wave measuring apparatus according to the first embodiment, and illustrate an operation of each portion and a gas flow path in the measurement sequence of the pulse wave measuring apparatus.

Referring to Fig. 3, after pressure pulse wave sensor 14 is attached to locate over the artery at the measurement position of the subject, a power switch included in manipulation portion 4 is switched on. Then, a not-shown power supply is turned on in step S1, CPU 1 starts operation, and the processing thereafter is started. CPU 1 reads the program recorded in ROM 2 and starts execution thereof

Immediately after turn-on of power, in step S2, CPU 1 carries out, for example, data check of ROM 2, operation check of RAM 3 and operation check of a peripheral circuit, and initial setting, in accordance with the program. In the initial state, it is assumed that three-port valve 10 has been switched to intake port 92 side of pump 9 (air tube 32 side).

In next step S3, CPU 1 outputs a control signal to open exhaust valve 7, to set air tubes 31 and 32 and the cuff pressure to the atmospheric pressure. Here, CPU 1 records a pressure applied to pressure sensor 11, that is, the atmospheric pressure, in RAM 3 by reading a pressure value digitized by A/D converter 5 via pressure sensor amplifier 12, and employs this pressure value as a reference pressure value for measurement of the cuff pressure subsequent to this pressure value.

Fig. 4 illustrates an operation of each portion in step S3, immediately after turn-on of power.

Referring to Fig. 4, immediately after turn-on of power, CPU 1 opens exhaust valve 7, leaving three-port valve 10 switched to intake port 92 side of pump 9 in the initial state. In addition, pump 9 remains stopped.

In step S3, the pressure applied to pressure sensor 11 at this time, that is, the atmospheric pressure, is digitized by A/D converter 5 via pressure sensor amplifier 12, read in CPU 1, and recorded in RAM 3. The pressure value recorded in RAM 3 is used as the reference pressure value for cuff pressure measurement subsequent to this pressure value. It is noted that exhaust valve 7 may have already been opened by the time of turn-off of the power.

When pressing of a measurement start switch included in manipulation portion 4 is detected in step S4, CPU 1 starts subsequent measurement sequence.

While the cuff pressure is at the atmospheric pressure, at the time of attachment of pressure pulse wave sensor 14, cuff 13 may sag vertically due to the weight of pressure pulse wave sensor 14 connected to cuff 13 or other components, or pressure pulse wave sensor 14 may be in contact with a skin surface of the subject due to unevenness of the measurement position of the subject.

Here, as data under no load of pressure pulse wave sensor 14 obtained at the time of start of measurement is used as a reference offset value for data subsequently obtained with pressure pulse wave sensor 14, pressure pulse wave sensor 14 preferably is not in contact with the skin surface of the subject at the time of start of measurement. Though it is possible to record data under no load of pressure pulse wave sensor 14 in ROM 2 when shipped from a factory, to read the data each time measurement is performed, and to use that data as the reference offset value, there is also the possibility of variation over time in the data under no load of pressure pulse wave sensor 14, or the possibility of such a phenomenon as offset drift. Accordingly, accuracy in measurement can be higher if the reference offset value for pressure pulse wave sensor 14 is obtained for each measurement. Therefore, when the measurement sequence is started, the following control is carried out in step S5.

Fig. 5 illustrates an operation of each portion and a gas flow path at the time of application of a negative pressure in step S5, when the measurement sequence is started.

As shown in Fig. 5, in step S5, CPU 1 closes exhaust valve 7. In addition, CPU 1 leaves three-port valve 10 switched to intake port 92 side of pump 9. Thereafter, CPU 1 operates pump 9.

Thus, the gas flow path from cuff 13 via air tubes 31 and 32 and intake port 92 and exhaust port 91 of pump 9 to air tube 30 is established, and the gas in air tubes 32 and 31 1 on intake port 92 side of pump 9 and the gas within cuff 13 are sent into air tube 30. Therefore, the internal pressure of air tubes 32 and 31 and the cuff pressure are set to a negative pressure equal to or lower than the atmospheric pressure, and the volume of cuff 13 decreases.

When the cuff pressure attains to a prescribed negative pressure value, CPU 1 stops pump 9. The volume of cuff 13 is set to a prescribed capacity, and accordingly pressure pulse wave sensor 14 connected to cuff 13 moves away from the skin surface of the subject.

In next step S6, the output of pressure pulse wave sensor 14 under no load is digitized in A/D converter 5 through multiplexer 15 and amplifier 16 for pressure pulse wave sensor, read in CPU 1, and recorded in RAM 3. The data recorded in RAM 3 is used as the reference offset value for pressure pulse wave sensor 14 as described above.

When the reference offset value for pressure pulse wave sensor 14 is obtained in step S6, pressing is started. Fig. 6 illustrates an operation of each portion and a gas flow path at the time of start of pressing in step S7.

As shown in Fig. 6, in step S7, CPU 1 opens exhaust valve 7. In addition, CPU 1 switches three-port valve 10 to exhaust port 91 side of pump 9. As a result of switching of three-port valve 10, air tube 30 on exhaust port 91 side of pump 9 is connected to air tube 31 and cuff 13. Thereafter, pump 9 is operated.

Thus, the flow path from the outside via exhaust valve 7, air tube 32, and intake port 92 and exhaust port 91 of pump 9 to air tubes 30 and 31 and cuff 13 is established, and the gas (air) is supplied to air tubes 30 and 31 and cuff 13. Therefore, the pressure of air tubes 30 and 31 and cuff 13 is increased.

As the cuff pressure increases, the volume of cuff 13 increases and pressure pulse wave sensor 14 comes in contact with the skin surface of the subject and thereafter it is pressed against the artery at the measurement position. During this period as well, the output of pressure pulse wave sensor 14 is digitized in A/D converter 5 via multiplexer 15 and amplifier 16 for pressure pulse wave sensor, read in CPU 1, and recorded in RAM 3.

In next step S8, CPU 1 finds an optimal level of pressing pressure pulse wave sensor 14 against the artery, for measuring the pulse wave. The method in step S8 is not limited to a particular method in the present invention, and any method may be adopted. For example, as a known technique, a method described in Japanese Patent Laying-Open No. 2004-313409 filed previously by the applicant of the subject application represents one specific example.

In subsequent steps S9 to S10, CPU 1 lowers the cuff pressure so as to attain an appropriate pressing value found in step S8.

Principles of operation for lowering the cuff pressure in steps S9 to S10 are the same as those of a method described, for example, in Japanese Patent Application No. 2003-333617 filed previously by the applicant of the subject application.

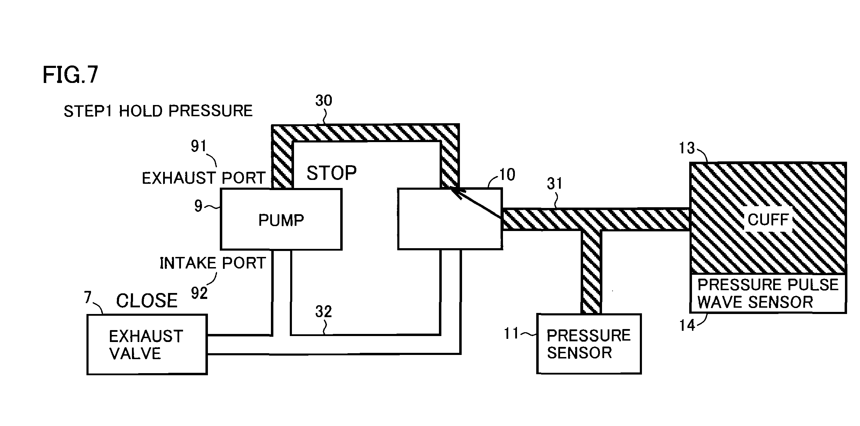

Figs. 7 to 10 illustrate an operation of each portion and a gas flow path in each step (STEP 1 to STEP 4) for lowering the cuff pressure in steps S9 and S10.

In STEP 1 (Fig. 7), CPU 1 closes exhaust valve 7. In addition, CPU 1 stops the operation of pump 9, leaving three-port valve 10 switched to exhaust port 91 side of pump 9. Therefore, air tubes 30 and 31 are closed and the internal pressure is held.

In STEP 2 (Fig. 8), CPU 1 opens exhaust valve 7, establishes the gas flow path from air tube 32 to the outside, and introduces the atmospheric pressure. Therefore, air tube 32 opens to the outside and the internal pressure is set to the atmospheric pressure.

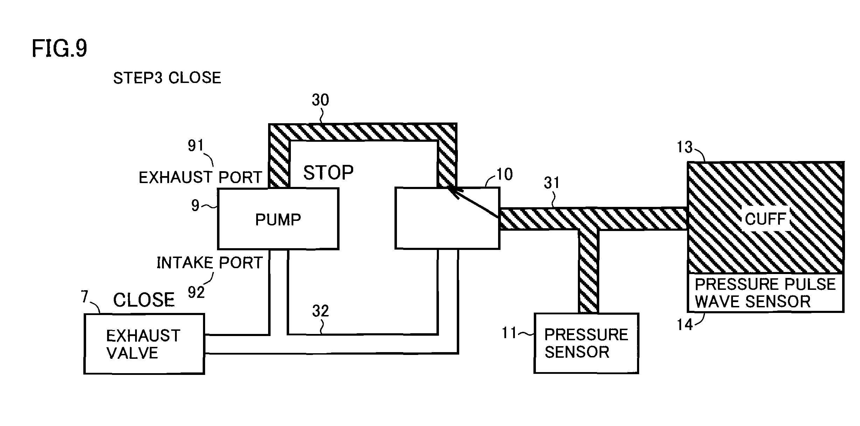

In STEP 3 (Fig. 9), CPU 1 closes exhaust valve 7. Therefore, air tube 32 is closed and the internal pressure is held at the atmospheric pressure.

In STEP 4 (Fig. 10), CPU 1 switches three-port valve 10 to intake port 92 side of pump 9 (air tube 32 side). Therefore, air tubes 32 and 31 and cuff 13 are connected and a closed state is established, and the internal pressure becomes uniform. Namely, the cuff pressure is reduced at a ratio of (volume of air tube 31 + volume of cuff 13)/(volume of air tube 32 + volume of air tube 31 + volume of cuff 13).

The pressure reduction operation in STEP 1 to STEP 4 above is repeated until the cuff pressure reaches the prescribed value determined in step S8 (step S 10). The pressure in air tubes 32 and 31 and cuff 13 becomes uniform through the operation above, however, the pressure in air tube 30 is held after STEP 1 of step S9. Therefore, pressure difference is created between the pressure in air tube 30 and the pressure in air tubes 32 and 31 and cuff 13. Accordingly, in lowering the cuff pressure, the operation in STEP 3 and STEP 4 (that is, switching operation of three-port valve 10) is preferably repeated so as to make the pressure in the tubes of the pulse wave measuring apparatus (air tubes 30, 32 and 31 and cuff 13) uniform.

In step S11, CPU 1 maintains the state in STEP 4 (Fig. 10) and holds the cuff pressure, and in step S12, the pressure pulse wave is measured. CPU 1 performs such processing as displaying the waveform of the pulse wave on display portion 6 in accordance with the measurement result, calculating a clinically significant pulse wave index such as Augmentation Index (hereinafter, abbreviated as AI), and displaying the calculated pulse wave index on display portion 6. Though AI is mentioned as an exemplary pulse wave index, the pulse wave index is not limited thereto.

After measurement in steps S11 and 12 is performed for a prescribed period of time, CPU 1 releases pressing against the artery by pressure pulse wave sensor 14 in step S13. Fig. 11 illustrates an operation of each portion and a gas flow path when the gas within cuff 13 is rapidly discharged and pressing by pressure pulse wave sensor 14 is released in step S13.

As shown in Fig. 11, in step S 13, CPU 1 opens exhaust valve 7. By opening exhaust valve 7 while leaving three-port valve 10 switched to intake port 92 side of pump 9, the gas flow path from cuff 13 via air tubes 31 and 32 and exhaust valve 7 to the outside is established and air tubes 31 and 32 and the cuff pressure are set to the atmospheric pressure. Therefore, pressing against the artery by pressure pulse wave sensor 14 is released. Thereafter, in addition, in a manner similar to step S5 above, the cuff pressure may be set to the atmospheric pressure or lower, so that pressure pulse wave sensor 14 connected to cuff 13 moves away from the skin surface of the subject.

Finally in step S 14, the final measurement result is displayed on display portion 6. Display content may be, for example, an average waveform of the pulse waves, an average value of AI values, or a value indicating fluctuation in measurement values (such as standard deviation), or a two-dimensional graph of these values and other indices (such as a blood pressure value), a graph of change over time that shows these values together with past data, a graph plotting a measurement value relative to a standard value of the index, and the like. It is noted that the display content is not limited to these specific examples.

A series of measurement sequences of the pulse wave measuring apparatus according to the present embodiment thus ends. The measurement sequence is by way of example, without limited thereto.

The pulse wave measuring apparatus according to the present embodiment is configured as shown in Fig. 1, so that two pumps 17 and 18 included in the configuration of the conventional pulse wave measuring apparatus (Fig. 21) can be replaced with a single pump 9 equivalent in performance and price. In addition, two three-port valves 20 and 21 included in the configuration of the conventional pulse wave measuring apparatus (Fig. 21) can be replaced with a single three-port valve 10 equivalent in performance and price.

According to such a configuration, as compared with the conventional pulse wave measuring apparatus, the volume of one pump and the volume of one three-port valve can be eliminated and reduction in size of the apparatus can be achieved.

In addition, according to such a configuration, as compared with the conventional pulse wave measuring apparatus, the price of one pump and the price of one three-port valve, both of which are particularly expensive parts, can be eliminated and reduction in cost of the pulse wave measuring apparatus can be achieved.

Moreover, according to such a configuration, the number of elements controlled by CPU 1 is reduced from five (pumps 17 and 18, the exhaust valve, and three-port valves 20 and 21) in the conventional pulse wave measuring apparatus to three (pump 9, exhaust valve 7, and three-port valve 10), so that the control circuit in the conventional pulse wave measuring apparatus can be replaced with a control circuit with a simplified configuration. Therefore, the effect of cost reduction can be enhanced.

### [Second Embodiment]

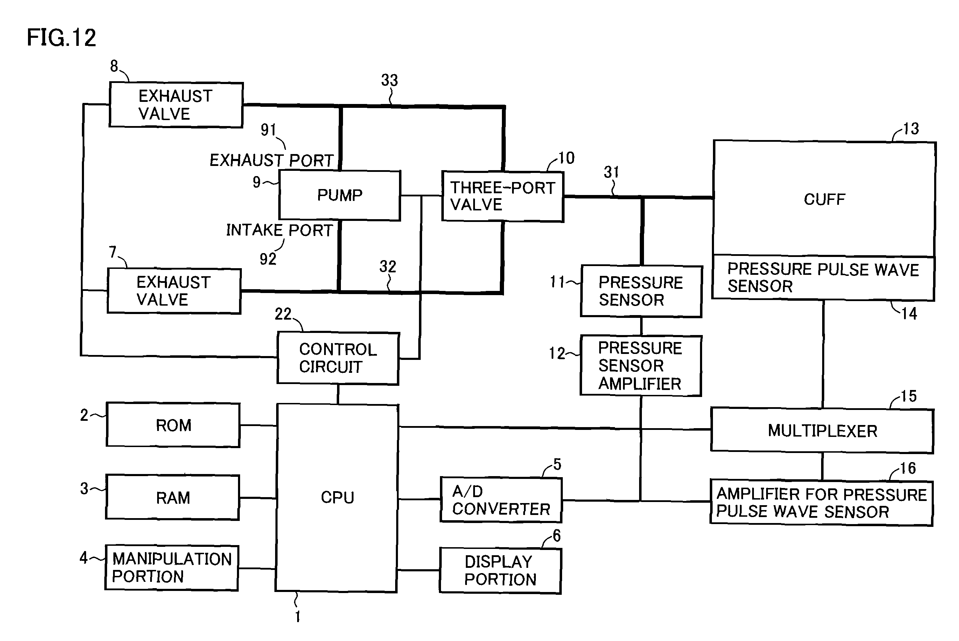

Fig. 12 illustrates a specific example of a configuration of a pulse wave measuring apparatus according to a second embodiment.

Referring to Fig. 12, the pulse wave measuring apparatus according to the second embodiment includes an exhaust valve 8 on exhaust port 91 side of pump 9, in addition to the configuration of the pulse wave measuring apparatus according to the first embodiment shown in Fig. 1, and exhaust valve 8, exhaust port 91 side of pump 9 and three-port valve 10 are connected via air tube 30.

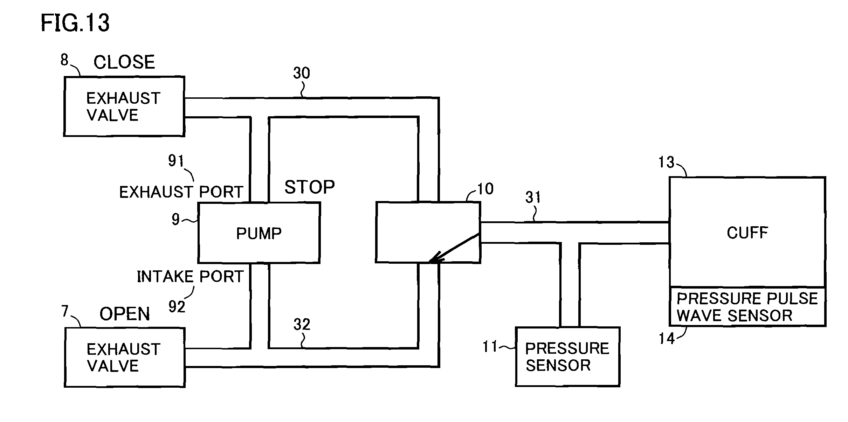

Figs. 13 to 20 are schematic diagrams illustrating a main portion of the pulse wave measuring apparatus according to the second embodiment, and illustrate an operation of each portion and a gas flow path in measurement sequence of the pulse wave measuring apparatus. It is noted that the measurement sequence in the pulse wave measuring apparatus according to the second embodiment is the same as that in the pulse wave measuring apparatus according to the first embodiment shown in Fig. 3.

Fig. 13 illustrates an operation of each portion in step S3, immediately after turn-on of power.

Referring to Fig. 13, immediately after turn-on of power, CPU 1 opens exhaust valve 7 and closes exhaust valve 8, leaving three-port valve 10 switched to intake port 92 side of pump 9 in the initial state. In addition, pump 9 remains stopped.

Fig. 14 illustrates an operation of each portion and a gas flow path at the time of application of a negative pressure in step S5, when the measurement sequence is started.

As shown in Fig. 14, in step S5, CPU 1 closes exhaust valve 7 and opens exhaust valve 8. In addition, three-port valve 10 is switched to intake port 92 side of pump 9. Thereafter, CPU 1 operates pump 9.

Thus, the flow path from cuff 13 via air tubes 31 and 32, intake port 92 and exhaust port 91 of pump 9, air tube 30, and exhaust valve 8 to the outside is established, so that the gas in air tubes 32 and 31 on intake port 92 side of pump 9 and the gas in cuff 13 are sent into air tube 30 and discharged to the outside. Therefore, the internal pressure of air tubes 32 and 31 and the cuff pressure attain to a negative pressure not higher than the atmospheric pressure and the volume of cuff 13 decreases.

Fig. 15 illustrates an operation of each portion and a gas flow path at the time of start of pressing in step S7.

As shown in Fig. 15, when the reference offset value for pressure pulse wave sensor 14 is obtained in step S6, CPU 1 opens exhaust valve 7 and closes exhaust valve 8 in step S7. In addition, three-port valve 10 is switched to exhaust port 91 side of pump 9. Thereafter, CPU 1 operates pump 9.

Thus, the flow path from the outside via exhaust valve 7, air tube 32, and intake port 92 and exhaust port 91 of pump 9 to air tubes 30 and 31 and cuff 13 is established, and the gas (air) is supplied to air tubes 30 and 31 and cuff 13. Therefore, air tubes 30 and 31 and cuff 13 are pressurized.

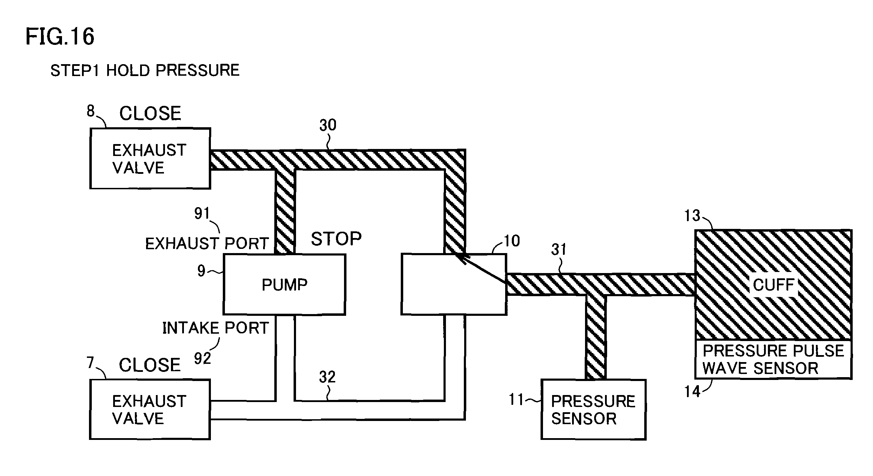

Figs. 16 to 19 illustrate an operation of each portion and a gas flow path in each step (STEP 1 to STEP 4) for lowering the cuff pressure in steps S9 and 10.

In STEP 1 (Fig. 16), CPU 1 closes exhaust valve 7. In addition, CPU 1 stops the operation of pump 9, leaving three-port valve 10 switched to exhaust port 91 side of pump 9. Therefore, air tubes 30 and 31 are closed and the internal pressure is held.

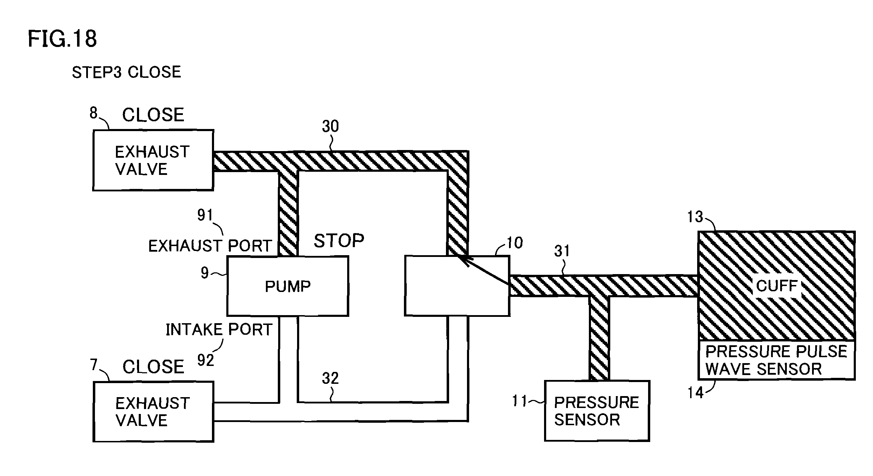

In STEP 2 (Fig. 17), CPU 1 opens exhaust valve 7, establishes the gas flow path from air tube 32 to the outside, and introduces the atmospheric pressure. Therefore, air tube 32 opens to the outside and the internal pressure is set to the atmospheric pressure. In STEP 3 (Fig. 18), CPU 1 closes exhaust valve 7. Therefore, air tube 32 is closed and the internal pressure is held at the atmospheric pressure.

In STEP 4 (Fig. 19), CPU 1 switches three-port valve 10 to intake port 92 side of pump 9 (air tube 32 side). Therefore, air tubes 32 and 31 and cuff 13 are connected and a closed state is established, and the internal pressure becomes uniform. Namely, the cuff pressure is reduced at a ratio of (volume of air tube 31 + volume of cuff 13)/(volume of air tube 32 + volume of air tube 31 + volume of cuff 13).

Fig. 20 illustrates an operation of each portion and a gas flow path when the gas within cuff 13 is rapidly discharged and pressing by pressure pulse wave sensor 14 is released in step S 13.

As shown in Fig. 20, in step S 13, CPU 1 opens exhaust valve 7. By opening exhaust valve 7 while leaving three-port valve 10 switched to intake port 92 side of pump 9, the gas flow path from cuff 13 via air tubes 31 and 32 and exhaust valve 7 to the outside is established, and air tubes 31 and 32 and the cuff pressure are set to the atmospheric pressure. Therefore, pressing against the artery by pressure pulse wave sensor 14 is released.

The pulse wave measuring apparatus according to the first embodiment is configured such that the gas in air tubes 32 and 31 and cuff 13 is sent into air tube 30 in step S5. Meanwhile, the pulse wave measuring apparatus according to the present embodiment is configured as shown in Fig. 12, so that the gas in air tubes 32 and 31 and cuff 13 is discharged to the outside via exhaust valve 8 in step S5. Namely, the pulse wave measuring apparatus according to the present embodiment is configured (Fig. 12) with an additional inexpensive exhaust valve in the configuration (Fig. 1) of the pulse wave measuring apparatus according to the first embodiment, so that load imposed on pump 9 at the time of application of a negative pressure can be lowered as compared with the pulse wave measuring apparatus according to the first embodiment.

### [Third Embodiment]

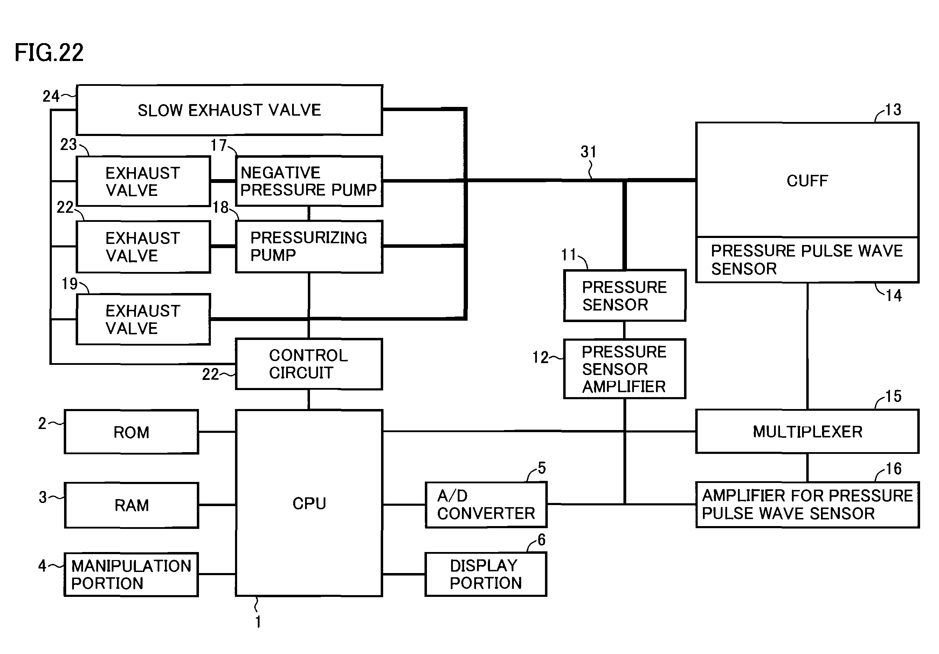

Fig. 22 illustrates a specific example of a configuration of a pulse wave measuring apparatus according to a third embodiment.

Referring to Fig. 22, the pulse wave measuring apparatus according to the third embodiment includes, as configuration for a measurement system, negative pressure pump 17, pressurizing pump 18, a slow exhaust valve 24, and an exhaust valve 26 each connected to cuff 13 via air tube 31, instead of exhaust valve 7, pump 9 and three-port valve 10 in the configuration of the pulse wave measuring apparatus according to the first embodiment shown in Fig. 1. In addition, exhaust valve 23 is connected to an exhaust side of negative pressure pump 17, and exhaust valve 23 is connected to an intake side of pressurizing pump 18.

Control circuit 22 is connected to negative pressure pump 17, pressurizing pump 18, slow exhaust valve 24, and exhaust valves 22, 23 and 26, and outputs a control signal to each portion. Exhaust valves 22, 23 and 26 are electromagnetic valves of which opening/closing is controlled by the control signal from control circuit 22.

Negative pressure pump 17 operates to introduce the gas in air tube 31 from the intake port and discharge the gas to the atmosphere through exhaust valve 23 from the exhaust port in response to the control signal input from control circuit 22. Pressurizing pump 18 operates to introduce the external gas from the intake port through exhaust valve 22 and discharge the gas from the exhaust port into air tube 31 in response to the control signal input from control circuit 22. Slow exhaust valve 24 operates to introduce the gas in air tube 31 from the intake port and gradually discharge the gas from the exhaust port to the atmosphere while adjusting an amount of discharge, in response to the control signal input from control circuit 22. Exhaust valve 22, exhaust valve 23, and exhaust valve 26 are directly connected to the intake port of pressurizing pump 18, the exhaust port of negative pressure pump 17, and air tube 31, respectively, and each operates to shut off/establish a flow path between the external gas and air tube 31 in response to the control signal input from control circuit 22.

Figs. 23 to 28 are schematic diagrams illustrating a main portion of the pulse wave measuring apparatus according to the third embodiment, and illustrate an operation of each portion and a gas flow path in the measurement sequence of the pulse wave measuring apparatus shown in Fig. 3.

In the pulse wave measuring apparatus according to the present embodiment, in step S3, CPU 1 outputs the control signal to open exhaust valve 26 and sets air tube 31 and the cuff pressure to the atmospheric pressure. Here, CPU 1 reads a pressure value obtained by digitizing a pressure applied to pressure sensor 11, that is, the atmospheric pressure, by using A/D converter 5 via pressure sensor amplifier 12, records the pressure value in RAM 3, and employs this pressure value as the reference pressure value for measurement of the cuff pressure subsequent to this pressure value. It is noted that exhaust valve 26 may have already been opened by the time of turn-off of the power. When the measurement start switch included in manipulation portion 4 is pressed in step S4, subsequent measurement sequence is started.

Fig. 23 illustrates an operation of each portion and a gas flow path in step S5, when a negative pressure is applied.

Referring to Fig. 23, in step S5, CPU 1 closes exhaust valves 22 and 26 and slow exhaust valve 24, opens exhaust valve 23, and operates negative pressure pump 17. Thus, the gas flow path from cuff 13 via air tube 31, the intake port and the exhaust port of negative pressure pump 17 and exhaust valve 23 to the outside is established, so that the gas remaining in cuff 13 set to the atmospheric pressure in step S3 is discharged to the atmosphere via air tube 31, the intake port and the exhaust port of negative pressure pump 17 and exhaust valve 23. Therefore, the internal pressure of air tube 31 and the cuff pressure are set to the negative pressure not higher than the atmospheric pressure and the volume of cuff 13 decreases.

When the cuff pressure attains to a prescribed negative pressure value, CPU 1 stops exhaust pump 17 and closes exhaust valve 23. The volume of cuff 13 is set to a prescribed capacity, and accordingly pressure pulse wave sensor 14 connected to cuff 13 moves away from the skin surface of the subject.

Fig. 24 illustrates an operation of each portion and a gas flow path in step S7, where pressurization is carried out.

Referring to Fig. 24, in step S7, CPU 1 opens exhaust valve 22 and operates pressurizing pump 18. Thus, the flow path from the outside via exhaust valve 22, the intake port and the exhaust port of pressurizing pump 18 and air tube 31 to cuff 13 is established, so that the gas is supplied to air tube 31 and cuff 13. Therefore, air tube 31 1 and cuff 13 are pressurized.

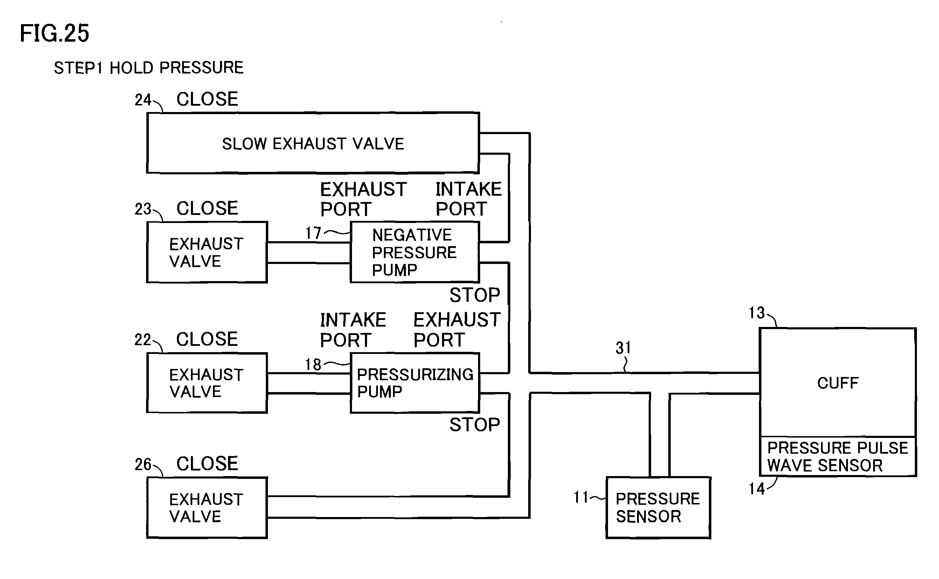

Figs. 25 to 27 illustrate an operation of each portion and a gas flow path in each step (STEP 1 to STEP 3) for lowering the cuff pressure in steps S9 and S10.

In STEP 1 (Fig. 25), CPU 1 closes all exhaust valves 22, 23 and 25 and slow exhaust valve 24, and stops negative pressure pump 17 and pressurizing pump 18. Therefore, air tube 31 is in a closed state and the internal pressure is held.

In STEP 2 (Fig. 26), CPU 1 opens slow exhaust valve 24 and establishes the gas flow path from cuff 13 via air tube 31 and slow exhaust valve 24 to the outside, so that the gas in cuff 13 is discharged to the outside. Here, CPU 1 can finely adjust a flow rate of the discharged gas based on on/off control or duty control of slow exhaust valve 24, so that pressure reduction of cuff 13 can finely be adjusted. It is noted that an exhaust valve with a simplified structure, capable of pressure reduction control in prescribed small pressure steps under on/off control, may be employed as slow exhaust valve 24, instead of a valve for exhaust.

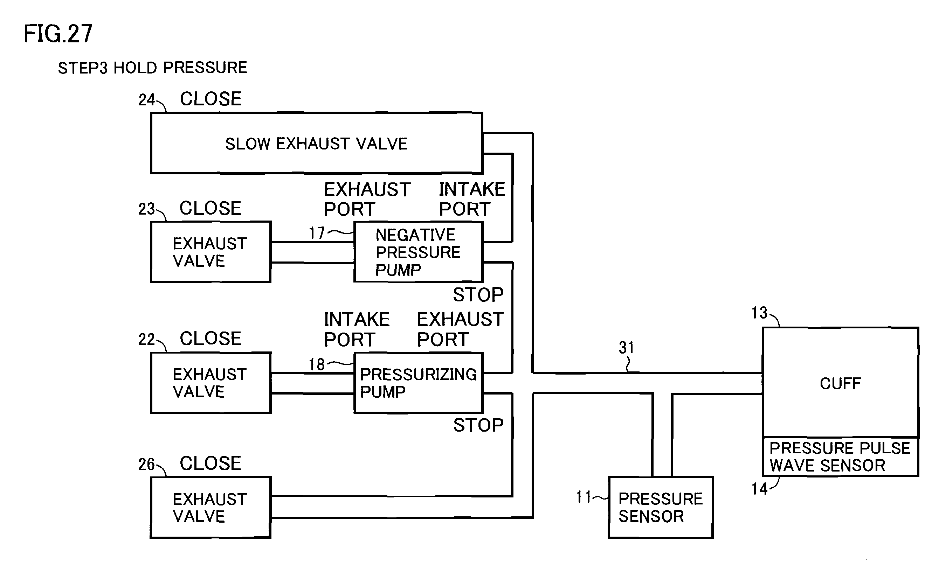

In STEP 3 (Fig. 27), CPU 1 closes all exhaust valves 22, 23 and 26 and slow exhaust valve 24, and stops negative pressure pump 17 and pressurizing pump 18. Therefore, air tube 31 is in a closed state and the internal pressure is held, as in STEP 1.

The pressure reduction operation in STEP 2 is repeated from the state where the internal pressure is held in STEP 3, until the cuff pressure attains to the prescribed value determined in step S8 (step S10).

In step S 11, CPU 1 holds the cuff pressure by maintaining the state in STEP 3 (Fig. 27), and measures the pressure pulse wave in step S12. After measurement in steps S 11 and 12 for a prescribed period of time, CPU 1 releases pressing against the artery by pressure pulse wave sensor 14 in step S13.

Fig. 28 illustrates an operation of each portion and a gas flow path when the gas within cuff 13 is rapidly discharged and pressing by pressure pulse wave sensor 14 is released in step S13.

Referring to Fig. 28, CPU 1 opens exhaust valve 26 in step S 13 and establishes the gas flow path from cuff 13 via air tube 31 and exhaust valve 26 to the outside, so that the gas in air tube 31 1 and cuff 13 is released to the outside and the internal pressure of air tube 31 1 and the cuff pressure are set to the atmospheric pressure. Therefore, pressing against the artery by pressure pulse wave sensor 14 is released.

### [Variation]

Depending on a type of an exhaust pump to be used, use thereof under such a condition that the pressure on the intake port side is lower than the pressure on the exhaust port side may be recommended. This is because, when the pressure of the intake port side is higher than the pressure on the exhaust port side, a check valve provided in the exhaust pump enters a backflow state and such backflow may be unfavorable depending on the structure of the exhaust pump.

In steps S7 to S12 above, the pressure on the intake port side of negative pressure pump 17 may be higher than the pressure on the exhaust port side thereof. In order to address such a situation, the volume between exhaust valve 23 and the exhaust port of negative pressure pump 17 is preferably minimized. By doing so, a backflow rate of the gas can be reduced and the problem as above can be prevented.

Alternatively, at the time of pressurization in step S7, CPU 1 preferably closes exhaust valve 23 and operates negative pressure pump 17. By doing so, the pressure between exhaust valve 23 and the exhaust port of negative pressure pump 17 can be higher than the pressure on the intake port side of negative pressure pump 17 and the problem as above can be prevented.

As described above, by using a component with a simplified structure and more inexpensive than a three-port valve instead of the three-port valve which is an expensive part, the pulse wave measuring apparatus according to the present embodiment is configured to connect the exhaust side of the pressurizing pump having the intake port side connected to an electromagnetic valve, the intake side of the exhaust pump having the exhaust port side connected to an electromagnetic valve, a flow path resistance connected to a control exhaust valve, and a quick exhaust valve to the cuff, as the configuration for attaining a function to avoid failure due to inadvertent protrusion of the pressure pulse wave sensor and/or the configuration for obtaining the reference offset value for the pressure pulse wave sensor. According to such a configuration, cost reduction as compared with the conventional configuration can be achieved.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

### INDUSTRIAL APPLICABILITY

The present invention can advantageously be used for suppressing manufacturing cost to achieve lower cost of a pulse wave measuring apparatus adopting a method of increasing pressing against an artery of a living body by a pressure pulse wave sensor by inflating an air bag to which the pressure pulse wave sensor is attached.

## Claims

1. A pulse wave measuring apparatus, comprising:
a pressure sensor (14) pressed against an artery of a living body;
a pressing portion (13) for pressing said pressure sensor;
a pulse wave measurement portion (1) measuring a pulse wave produced from said artery based on pressure information output from said pressure sensor in a process of variation in a level of pressing said pressure sensor by said pressing portion;
a pressing adjustment portion (30, 31, 32) including a gas accommodation portion and adjusting said level of pressing said pressure sensor by using a pressure of gas in said gas accommodation portion;
a supply portion (9, 18) supplying gas to said gas accommodation portion; and
a flow path control portion (22) including a valve (7, 22, 23) for taking in and discharging the gas between said gas accommodation portion and outside by opening and for shutting off intake and discharge of the gas between said gas accommodation portion and the outside by closing, and adjusting a gas flow path between the outside and said gas accommodation portion.

2. The pulse wave measuring apparatus according to claim 1, further comprising an exhaust portion (17) discharging the gas in said gas accommodation portion (31) to the outside; wherein
said valve includes a first valve (22) connected to an intake side where the gas outside said supply portion (18) is taken in and a second valve (23) connected to an exhaust side where the gas is discharged to the outside of said exhaust portion, and
said flow path control portion adjusts the gas flow path between the outside and said gas accommodation portion by controlling opening/closing of said first valve and said second valve and an operation of said supply portion and said exhaust portion.

3. The pulse wave measuring apparatus according to claim 2, wherein
in moving said pressure sensor away from a surface of said living body by means of said pressing adjustment portion, said flow path control portion controls said supply portion to stop operation and said exhaust portion to operate and controls said first valve to close and said second valve to open, so that a first flow path from said gas accommodation portion via said exhaust portion and said second valve to the outside is established and the gas in said gas accommodation portion is discharged.

4. The pulse wave measuring apparatus according to claim 2, wherein
in pressing said pressure sensor with said pressing portion by means of said pressing adjustment portion, said flow path control portion controls said supply portion to operate and controls said first valve to open and said second valve to close, so that a second flow path from the outside via said first valve and said supply portion to said gas accommodation portion is established and the gas is supplied to said gas accommodation portion.

5. The pulse wave measuring apparatus according to claim 4, wherein
in pressing said pressure sensor with said pressing portion by means of said pressing adjustment portion, said flow path control portion further controls said exhaust portion to operate.

6. The pulse wave measuring apparatus according to claim 2, further comprising a slow exhaust portion (24) discharging the gas in said gas accommodation portion to the outside while adjusting an amount of discharge; wherein
in adjusting said level of pressing said pressure sensor by means of said pressing adjustment portion, said flow path control portion controls said supply portion and said exhaust portion to stop operation and said slow exhaust portion to operate and controls said first valve and said second valve to close, so that a third flow path from said gas accommodation portion via said slow exhaust portion to the outside is established and the gas in said gas accommodation portion is discharged.

7. The pulse wave measuring apparatus according to claim 6, wherein
said flow path control portion repeats establishment of said third flow path and closing of said gas accommodation portion until an internal pressure of said gas accommodation portion attains to a prescribed pressure.

8. The pulse wave measuring apparatus according to claim 2, wherein
said valve further includes a third valve (19) connected to said gas accommodation portion, and
in releasing said pressure sensor from said living body by means of said pressing adjustment portion, said flow path control portion controls said supply portion and said exhaust portion to stop operation and controls said first valve and said second valve to close and said third valve to open, so that a fourth flow path from said gas accommodation portion via said third valve to the outside is established and the gas in said gas accommodation portion is discharged.

9. The pulse wave measuring apparatus according to claim 1, wherein
said gas accommodation portion includes a first gas accommodation portion (31), a second gas accommodation portion (32) and a third gas accommodation portion (33) having a prescribed capacity,
said pressing adjustment portion adjusts said level of pressing said pressure sensor by using the pressure of the gas in said first gas accommodation portion,
said supply portion (9) is provided between said second gas accommodation portion and said third gas accommodation portion and supplies the gas to said second gas accommodation portion by taking in the gas in said third gas accommodation portion and discharging the gas to said second gas accommodation portion,
said valve includes a first valve (7) for taking in and discharging the gas between (7) said third gas accommodation portion and the outside by opening and for shutting off intake and discharge of the gas between said third gas accommodation portion and the outside by closing, and
said flow path control portion further includes a switching portion (10) switching between a first connection state where said first gas accommodation portion is connected to an exhaust side of said supply portion via said second gas accommodation portion and a second connection state where said first gas accommodation portion is connected to an intake side of said supply portion via said third gas accommodation portion, and adjusts a gas flow path among said first gas accommodation portion, said second gas accommodation portion, and said third gas accommodation portion.

10. The pulse wave measuring apparatus according to claim 9, wherein
said switching portion is a three-port valve having a first port connected to said first gas accommodation portion, for taking in and discharging the gas, a second port connected to said second gas accommodation portion, for taking in and discharging the gas, and a third port connected to said third gas accommodation portion, for taking in and discharging the gas.

11. The pulse wave measuring apparatus according to claim 10, wherein
in releasing said pressure sensor from a surface of said living body by means of said pressing adjustment portion, said flow path control portion controls said three-port valve to connect said first port to said third port to attain said second connection state, controls said supply portion to operate, and controls said first valve to close, so that a first flow path from said first gas accommodation portion via said third gas accommodation portion to said second gas accommodation portion is established and the gas in said first gas accommodation portion is discharged.

12. The pulse wave measuring apparatus according to claim 10, wherein
in pressing said pressure sensor with said pressing portion by means of said pressing adjustment portion, said flow path control portion controls said three-port valve to connect said first port to said second port to attain said first connection state, controls said supply portion to operate, and controls said first valve to open, so that a second flow path from the outside via said third gas accommodation portion and said second gas accommodation portion to said first gas accommodation portion is established and the gas is supplied to said first gas accommodation portion.

13. The pulse wave measuring apparatus according to claim 10, wherein
in adjusting said level of pressing said pressure sensor by means of said pressing adjustment portion, said flow path control portion controls said three-port valve to connect said first port to said second port to attain said first connection state, controls said supply portion to stop said operation, and controls said first valve to open, so that a third flow path from said third gas accommodation portion to the outside is established and an internal pressure of said third gas accommodation portion attains to an atmospheric pressure, and thereafter, after closing said first valve and closing said third gas accommodation portion, said flow path control portion controls said three-port valve to connect said first port to said third port to attain said second connection state and controls said supply portion to stop said operation, so that a fourth flow path from said first gas accommodation portion to said third gas accommodation portion is established and an internal pressure of said first gas accommodation portion is reduced.

14. The pulse wave measuring apparatus according to claim 13, wherein
said flow path control portion repeats establishment of said third flow path, closing of said third gas accommodation portion, and establishment of said fourth flow path until the internal pressure of said first gas accommodation portion attains to a prescribed pressure.

15. The pulse wave measuring apparatus according to claim 10, wherein
in releasing said pressure sensor from said living body by means of said pressing adjustment portion, said flow path control portion controls said three-port valve to connect said first port to said third port to attain said second connection state, controls said supply portion to stop said operation, and controls said first valve to open, so that a fifth flow path from said first gas accommodation portion via said third gas accommodation portion to the outside is established and the gas in said first gas accommodation portion is discharged.

16. The pulse wave measuring apparatus according to claim 10, wherein
said valve further includes a second valve (8) for taking in and discharging the gas between said second gas accommodation portion and the outside by opening and for shutting off intake and discharge of the gas between said second gas accommodation portion and the outside by closing, and
in moving said pressure sensor away from a surface of said living body by means of said pressing adjustment portion, said flow path control portion controls said three-port valve to connect said first port to said third port to attain said second connection state, controls said supply portion to operate, controls said first valve to close, and controls said second valve to open, so that a sixth flow path from said first gas accommodation portion via said third gas accommodation portion and said second gas accommodation portion to the outside is established and the gas in said first gas accommodation portion is discharged.
